# EUROPEAN PATENT SPECIFICATION

(11) **EP 1 811 904 B1**
(45) Date of publication and mention of the grant of the patent: **26.08.2009**
(21) Application number: 05806429.6
(22) Date of filing: 16.11.2005
(51) Int. Cl.: A61B 17/04, A61B 17/064

(54) **TISSUE ANCHOR**
GEWEBEANKER
ANCRAGE DE TISSU

(30) Priority: 17.11.2004 GB 0425296
(43) Date of publication of application: 01.08.2007
(73) Proprietor: GRAMPIAN HEALTH BOARD, Forresterhill House Forresterhill Aberdeen AB25 2ZB (GB); The Robert Gordon University, Aberdeen, AB9 1FR (GB)
(72) Inventor: JOHNSTONE, Alan, John, Aberdeen AB15 9AD (GB)
(74) Representative: Brown, James Douglas
(86) International application number: PCT/GB2005/004409
(87) International publication number: WO 2006/054071

(56) References cited:
- EP-A- 1 033 115
- US-A- 5 002 562
- US-A1- 2002 019 636
- US-A1- 2003 033 006
- US-A1- 2003 045 893

## Description

The present invention relates to a tissue anchor, particularly for use in bone, cartilage and other tissues of a body.

Different medical devices for use in vivo are known, such as the surgical clip disclosed in US5002562 or spinal staple disclosed in US2002/0019636.

Tissue anchors are widely used in surgery to retain and anchor sutures or other restraining devices. Present designs generally employ asymmetric anchors that are inserted into drilled holes in a bone. In some cases anchors are provided with threads to engage within a threaded hole, or rely on their asymmetric designs for anchorage within the bone or other tissue. Existing designs of tissue anchors are generally bulky, which limits their usefulness in certain bones, e.g. hands and feet.

US2003/0033006 discloses a device for the repair of arteries and EP1033115 discloses a ligament fixation device. Both of these devices offer little resistance to withdrawal from the tissue into which they are inserted.

According to the invention there is provided a tissue anchor in accordance with claim 1.

The sharp leading end or point on the first portion is sharpened to penetrate the dense tissue when the first and second portions are held in a first configuration relative to one another. Once the point has penetrated the tissue e.g. bone, the resilient device applies a force between the first and second portions in order to move them into a second configuration that is adapted to resist withdrawal from the tissue in the opposite direction of penetration.

Typically, the tissue anchor has a barb pointing in the opposite direction to the main point on the first portion. In preferred embodiments, the first and second portions are mirror image parts with forward facing points and corresponding barbs that extend in a different direction.

In a preferred format, each portion is generally in the form of a hook with a forward pointing end to penetrate the tissue, sloping back to a rearward pointing barb at the trailing end. The or each barb typically extends the tissue, sloping back to a rearward pointing barb at the trailing end. The or each barb typically extends radially outward further than the points at the leading end.

The resilient device can simply be a resilient wire or piece of sprung plastics material or metal such as sprung steel connecting the two hooks. The resilient device biases the two portions apart from one another in the absence of any force.

When the device is to be delivered, the two portions are typically forced toward one another against the natural bias of the resilient device before the tissue anchor is delivered into the tissue. In preferred embodiments of the device with the double hook configuration, this forces the tips of the respective hooks on the first and second portions towards one another so that they meet at their leading edge points, and the force applied typically maintains them in this first configuration while the tissue anchor is being delivered. Once the tissue anchor has been delivered to the desired location in the tissue, the force maintaining the device in the first configuration is removed, and in the preferred embodiments, the resilient member then splays apart the points at the leading ends of the first and second portions, and also moves the barbs further apart in order to lodge the tissue anchor securely within the tissue under the force applied by the resilient device.

Optionally, the tissue anchor can be twisted around its axis of insertion, so as to lodge the barbs and points more firmly within the tissue.

In some embodiments, the tissue anchor is forced into the closed configuration for delivery (with the leading edge points of the hooks forced together as described above) by threading a suture through the tissue anchor and threading the suture through a delivery sleeve, and then pulling the suture and the tissue anchor relative to the delivery sleeve so that the sleeve slides along the suture and applies a force to the tissue anchor. This force applied by the delivery sleeve typically moves the first and second portions from their naturally splayed open configuration to their closed configuration with the leading edge points forced together. The pressure applied via the suture and the delivery sleeve typically maintains the tissue anchor in the closed configuration all the way through the delivery process, and one advantage of this is that the tissue anchor can be hammered into place using the delivery sleeve as an anvil.

In some other embodiments the tips of the hooks are pressed together as outlined above by the action of the resilient device, which can be forced into a suitable configuration to achieve this by the confines of the delivery sleeve. For example, some of the resilient devices according to this embodiment can be generally teardrop shaped in the form of a loop with the hooks connected at the narrowed end. In such embodiments, the loop part of the drop is compressed by the confines of the delivery sleeve, so as to push the tips together. This kind of embodiment avoids the need to pull the suture or to apply other external force to it to keep the tips of the hooks together during insertion.

Once the tissue anchor is in the required position, the delivery sleeve can simply be withdrawn from the tissue, leaving the tissue anchor fixed in the correct position, and the suture already trailing out of the required path.

Typically the diameter of the delivery sleeve is less than the diameter of the barbs of the tissue anchor when in the closed configuration. This allows withdrawal of the delivery sleeve without disturbing the tissue anchor, as the rearward facing barbs can bite into solid bone beyond the nominal diameter of the aperture in the tissue made by the delivery sleeve.

If the tissue anchor is to be withdrawn from the tissue, it can be forced into the closed configuration for withdrawal by insertion of a larger diameter recovery sleeve, either over the delivery sleeve or simply over the suture. The diameter of the recovery sleeve is typically wider than the diameter of the splayed barbs in the second configuration, so that during withdrawal of the tissue anchor from the tissue, the rearward facing barbs no longer impede withdrawal of the tissue anchor.

A leading edge of the recovery sleeve can be provided with cutting formations to cut a hole in the tissue and thereby facilitate removal of the sleeve. An internal surface of the recovery sleeve can be provided with an annular groove to accommodate the laterally outermost barbs on the tissue anchor.

The tissue anchor of the invention facilitates a method of setting an anchor in tissue, the method comprising the steps of:
forcing the first and second portions into a first configuration in which the tissue anchor stores energy in the resilient device;
inserting the at least one provided on the first portion into the tissue; and
anchoring the tissue anchor within the tissue by removing the force on the first and second portions to allow the first and second portions to occupy the open configuration in which the first and second portions are spaced relative to one another.

The above method can include the step of penetrating dense tissue with the point of the first portion.

The method can also include urging the first and second portions into the first configuration against the bias of the resilient device prior to inserting the tissue anchor into the tissue. The method can include maintaining the first and second portions in the first configuration during insertion of the tissue anchor into the tissue.

The method can include introducing the tissue anchor into a delivery sleeve, which forces the tissue anchor into the second configuration. The method can include withdrawing the delivery sleeve from the tissue anchor and permitting the tissue anchor to change from the first configuration to the second configuration.

Once the tissue anchor of the present invention has been disposed in a tissue, the tissue anchor can be recovered by the following method. The recovery method comprises the steps of urging the first and second portions into the first configuration in which the first and second portions are urged against the bias of the resilient device, engaging the tissue anchor within a recovery sleeve and recovering the tissue anchor by withdrawing the recovery sleeve.

The method can include introducing the tissue anchor into a sleeve having a diameter less than the lateral extent of hooks provided on each of the first and second portions and urging the sleeve against a rear face of each hook to thereby urge the first and second portions against the bias of the resilient device.

The method can include accommodating a suture coupled to the tissue anchor in a throughbore of the sleeve. The method can also include sliding the sleeve along the anchored suture and thereby guiding the sleeve through the tissue to the tissue anchor to facilitate removal or adjustment thereof.

The method can also include accommodating the sleeve and the tissue anchor within the recovery sleeve before withdrawing the recovery sleeve and recovering the tissue anchor.

The method can also include retaining the tissue anchor within the bore of the recovery sleeve by providing an annular groove on an internal surface of the recovery sleeve and engaging the laterally outermost portion of the hooks in the groove.

The method can further include cutting through tissue using a leading end of the recovery sleeve to access the tissue anchor prior to its recovery.

An embodiment of the present invention will now be described by way of example, and with reference to the accompanying drawings, in which:-
Fig. 1 shows a tissue anchor in an open configuration;
Fig. 2 is a side view of a tissue anchor in an open configuration in which the rearward facing barbs resist withdrawal of the tissue anchor from a body with a suture attached;
Fig. 3 is a side view of the Fig. 1 tissue anchor with a delivery sleeve;
Fig. 4 is a close up view of the Fig. 2 tissue anchor and sleeve in the closed configuration with force applied via the delivery sleeve and suture to close the points of the tissue anchor;
Fig. 5 is a side view similar to Fig. 3, but showing the removal of force applied via the delivery sleeve and suture, and the splaying of the points of the tissue anchor as a result of force applied by the resilient device;
Fig. 6 is a side view similar to Figs. 2 to 4, showing the tissue anchor being closed by force applied by a suture and a recovery tube;
Fig. 7a and b are side views of a second embodiment of a tissue anchor showing the anchor in its closed configuration for insertion (a) and in the open configuration (b) after withdrawal of the delivery sleeve;
Fig. 8 is a perspective view of a third embodiment of a tissue anchor;
Fig. 9 is a plan view of the Fig. 8 embodiment;
Fig 10 is a side view of a further embodiment of a tissue anchor; and
Fig 11 is a side view of a further embodiment of a tissue anchor.

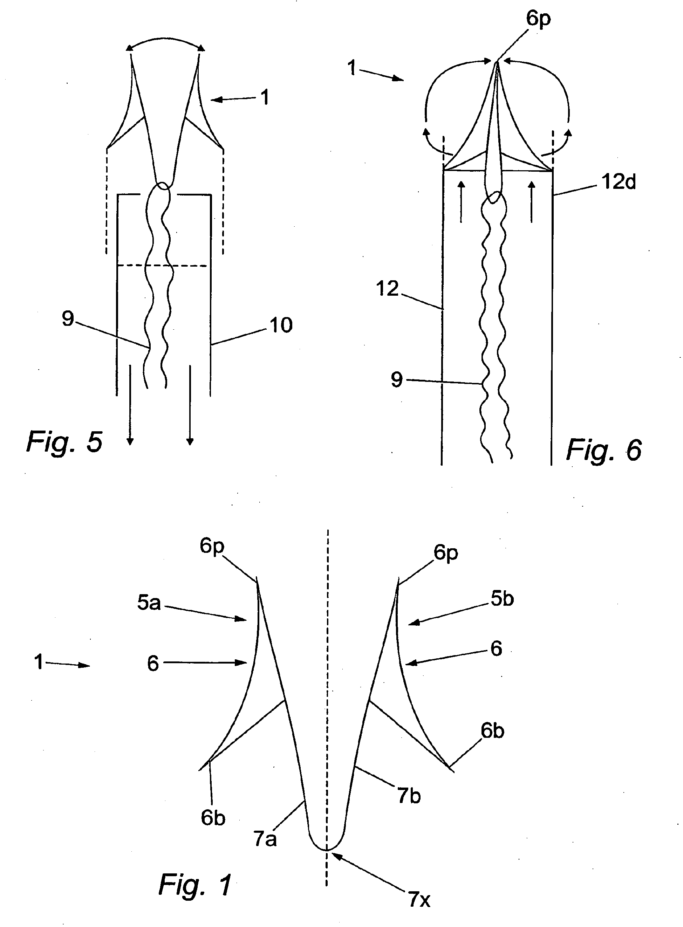

Referring now to the drawings, Fig. 1 shows a side view of a tissue anchor 1 having first and second portions in the form of arms 5a and 5b. Each arm 5 comprises a hook portion 6 having a sharpened point 6p at the leading end of the anchor 1, and a barb 6b pointing away from the leading end. The hook portion 6 is optionally smoothly curved between the barbs 6b and point 6p, so that the tip of the barb 6b is spaced radially outwards from the point 6p. The lower surface 61 of the hook 6 is canted at an angle of less than 90° with respect to the arm, so that the barbs 6b on each hook portion points away from the leading edge of the device (at the point 6p).

Each arm is formed from a stiff wire or steel strip. In the embodiments shown in the drawings, the two arms are made from a single continuous flexible steel strip or wire, but it would be acceptable to form the arms separately, either from a resilient material or a rigid material, and connect them by a resilient device such as a leaf spring, a hinge or some other device.

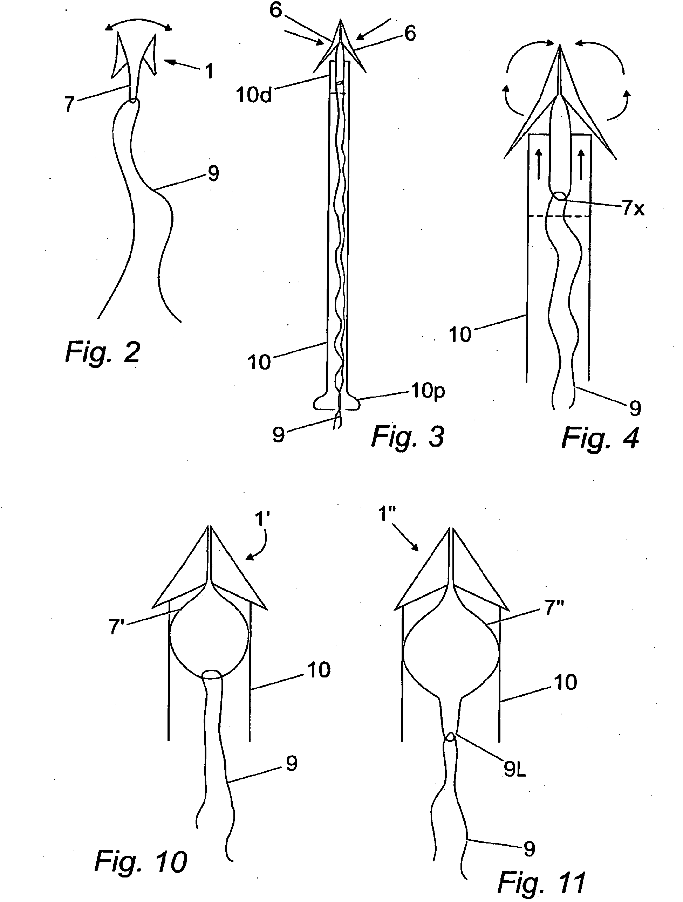

Referring now to Fig. 2, the arms are connected in a general V-shape with the resilient device at the apex 7x of the V. Other shapes could be used apart from V-shapes. In the embodiment shown, the two arms are formed from a continuous piece of sprung steel, to the ends of which the hooks are attached as shown. The apex 7x of the V serves as the resilient device and the arms are thereby biased apart into the general V-shape shown in Figs. 1 and 2 in the resting position. The inherent resilience of the sprung steel strip allows the tissue anchor to flex as shown in Fig. 2 when force is applied to it, and returns the tissue anchor to the open configuration shown in Figs. 1 and 2 when the force is removed.

Each of the hooks 6 has three sides in the embodiments shown. One side extending between the point 6p and the barb 6b is generally arcuate and adopts a slightly concave configuration. The concave configuration assists in the passage of the hook through the tissue, but a straight side would suffice in this embodiment. Another side of the hook 6 connects the hook to the end of the arm 7. The end of the arm 7 is generally resistant to flex, either as a result of the connection of the hook at that point, or by being made of an inherently stiffer material at the end of the arm. The remaining lower side 61 of the hook extends between the barb 6b and the arm 7. The angle made between this lower side 61 of the hook 6 and the arm 7 is generally less than 90°, so that the barb 6b faces away from the leading end at the point 6p, and towards the trailing end at the apex 7x between the arms 7. An angle of 90° in this instance would also suffice, but the acute angle made by the rearward facing lower side 61 of the hook enables the barbs 6b to anchor more effectively within the tissue after insertion, and facilitates loading of the device to push the tips 6p together by loading the delivery sleeve.

In use, the natural position of the tissue anchor 1 is as shown in Fig. 1 and Fig. 2, with the points 6p of the hooks 6 splayed apart by the natural resilience of the sprung steel in the arms 7. A suture 9 is threaded between the arms 7a, 7b as shown in Fig. 2 and the free ends of the suture are then gathered and inserted into a distal end 10d of a delivery tube 10, the free ends of the suture 9 being recovered from the proximal end 10p of the delivery tube 10, as shown in Fig. 3. The suture 9 can then be pulled through the proximal end 10p of the delivery tube 10. This pulls the apex 7x and the arms 7a, 7b down into the delivery tube, and pulls the lower sides 61 of the hooks against the proximal end 10b of the delivery tube 10. This forces the points 6p on each hook together as shown in Fig. 4. In the embodiments shown in Fig. 3 and Fig. 4, the outer diameter of the delivery tube 10 is less than the distance between the barbs 6b in the closed position shown in Fig. 4, so that the ends of the barbs 6b protrude radially beyond the outer diameter of the delivery tube 10, and the outer diameter of the delivery tube 10 presses against the middle of the rear facing lower side 61 of the hook 6.

Once the suture 9 has been pulled tight through the delivery tube 10, and the points 6p of the leading end of the tissue anchor 1 are closed together as shown in Fig. 3 and Fig. 4, the tissue anchor is then inserted into the body while the tension on the suture 9 is maintained, thereby keeping the points 6p of the hook together. The tissue anchor 1 can be inserted either by simply pushing the delivery sleeve into the tissue, or by hammering the proximal end 10p of the delivery sleeve. Once the tissue anchor 1 is in the required position as judged by the angle and depth of insertion of the delivery sleeve into the tissue, the assembly can then optionally be forcibly rotated around the axis of the delivery sleeve 10 to twist the barbs 6b in one or both rotational directions. This twisting movement moves the barbs 6b out of the path that the hook 6 has cut through the tissue during insertion, and tends to lodge the barbs 6b in sound bone or other tissue thereby further resisting withdrawal of the tissue anchor 1 therefrom.

At that point, the tension applied to the suture 9 relative to the delivery sleeve 10 can be removed, allowing removal of the delivery sleeve 10, and leaving the tissue anchor 1 firmly lodged in the tissue. When the tension is removed from the suture 9 and the distal end 10d is withdrawn from the hooks 6, the natural resilience of the resilient device on the tissue anchor 1 splays the arms 7a, 7b apart from one another, and moves the hooks 6, and thus the barbs 6b further apart as shown in Fig. 5, thereby further embedding the tissue anchor within the tissue, and resisting withdrawal. The delivery sleeve 10 can be withdrawn over the suture 9, leaving the suture 9 in place in the path cut by the tissue anchor 1 during insertion. The suture can then be fastened to another tissue anchor, or to other implant devices or fastened to other tissues as necessary.

If the tissue anchor is to be removed, either at completion of treatment, or because of incorrect placement, the delivery sleeve 10 can be reinserted to close the tissue anchor 1 and permit withdrawal, but in preferred embodiments, removal is facilitated by a separate recovery sleeve 12 as shown in Fig. 6.

The recovery sleeve 12 typically has a wider outer diameter than the delivery sleeve 10, and when placed over the suture 9 (with or without the delivery sleeve being present) and pushed against the tissue anchor 1, the distal end 12d of the recovery sleeve 12 abuts against the tips of the barb 6b, and the outer diameter of the recovery sleeve 12 is typically larger than the distance between the barbs 6b while in the closed position. Therefore, the rearward facing barbs 6b can be shielded by the recovery sleeve 12 so that they do not impede withdrawal from the tissue when engaged by the recovery sleeve 12. The suture 9 is optionally tensioned relative to the recovery sleeve 12 in the same way as described for the delivery sleeve 10 in order to close the points 6p of the hook 6, so that the tissue anchor 1 can then be withdrawn from the tissue without barbs etc causing damage to the tissue during the withdrawal process. The angle made between the rear facing side of the hook 6 and the arm 7 is typically sufficiently acute so as to create an acute angle in both open and closed configurations of the device. Suitable angles can be 50° to 80°. In some embodiments of the device, the recovery sleeve 12 can have cutting formations such as bevelled or chiselled leading edges on its distal tip to enlarge holes made by the insertion of the anchor. The delivery sleeve may also optionally have an annular groove in the distal end to accommodate the radially outermost edges of the hooks.

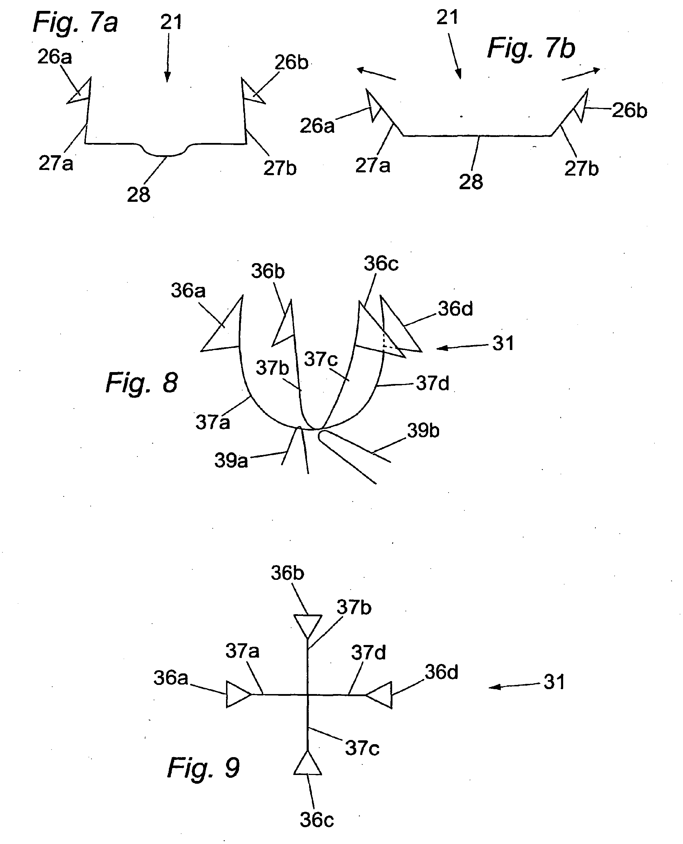

Fig. 7 shows a modified embodiment of a tissue anchor 21 in the form of a staple, with hooks 26, arms 27, and a bar 28 connecting the two arms. The bar 28 can be sprung to provide the resilient device, and can take the form of a leaf spring, or simply a section of sprung steel can be provided to form the connection between the arms 27 and the bar 28. The resilient device(s) biases the arms 27 into the open position shown in Fig. 7b, and the tissue anchor 21 can be inserted in the closed configuration shown in Fig. 7a as described for the previous embodiment, by using a delivery sleeve to force the arms 27 into the closed configuration.

Fig. 8 shows a further embodiment of a tissue anchor 31 having hooks 36, and arms 37 as previously described. The embodiment 31 differs from the first embodiment described in that instead of two hooks and two arms it has at least four hooks 36a, b, c, d, each located on a respective arm 37a, b, c, and d. This can be achieved simply by connecting two tissue anchors 1 together by welding the apices together in a cruciform arrangement. It will be appreciated that the tissue anchor 31 shown in Figs. 8 and 9 can have 3, 4, 5, 6, 7 or any other number of respective arms and hooks, and the example shown is merely illustrative of the principle and is not intended to be limiting. One advantage of the embodiment shown in Fig. 31 is that separate sutures 39a and 39b can be attached to different parts of the arms 37. For example, if the embodiment 31 is considered as a pair of tissue anchors formed from a first tissue anchor comprising arms 37a, 37d and hooks 36a, 36d, and a second tissue anchor comprising arms 37b, 37c and hooks 36b, 36c, wherein the apices of the tissue anchors are connected together by welding, gluing or some other kind of connection then the sutures 39a and b can be looped over any of four different positions, for example over any of the arms 37a, b, c or d. This means that a number of different sutures can be attached to a single tissue anchor 31 without the risk of the sutures interfering with one another. This is extremely useful, because when tension is applied to the suture after the tissue anchor has been lodged within the tissue, it can often happen in prior art devices that the tensioning of one suture causes another suture attached to the same tissue anchor to be trapped by the tensioning of the first suture, which can lead to the surgeon thinking that the second suture has been tied firmly when in fact it is not, and importantly can impede the free running of the second suture, which interferes with knotting techniques. The provision of separate and discreet attachment points provided by the separate arms 37a, b, c and d means that multiple sutures can be attached to the single tissue anchor 31 with a reduced risk of fouling one another during tensioning. The tissue anchor 31 shown here can take up to four separate sutures, one looped over each of arms 37a,b,c and d, but other multiples are clearly within the scope of the invention.

Fig 10 shows a further embodiment of a tissue anchor 1', which has arms 7' formed together and compressed into a general teardrop shape when the device 1' is in a closed configuration and held within the bore of the delivery sleeve 10. The arms 7' comprise sprung steel and are kept in the closed configuration by the pressure of the delivery sleeve acting on the loop portion of the arms 7'. As the hooks are connected to the arms at the narrowed end of the teardrop shape, the tips of the hooks are pressed together during insertion into the body by the force exerted on the arms by the inner surface of the bore of the sleeve, and this embodiment requires no additional force to keep it in the closed configuration.

Fig 11 shows a similar embodiment of a tissue anchor 1", which also has arms 7" formed together and compressed into a general teardrop shape when the device 1" is in a closed configuration and held within the bore of the delivery sleeve 10, similar to the Fig 10 embodiment. This embodiment 1'' also has a suture loop 91 at the proximal end of the arms 7" to restrict slippage of the suture around the arms 7".

The Fig 10 and 11 embodiments also place less train on the suture, as they avoid pinching the very thin and fragile suture at the apex of the arms during tensioning.

Modifications and improvements can be incorporated without departing from the scope of the invention. For example, in some configurations, the hooks 6 can be planar as shown in Figs. 1 to 6, or can be plough-shaped as shown in Figs. 8 and 9. Other forms of hook can be adopted if desired. In certain embodiments, the insertion sleeve 10 can be provided with graduations (e.g. laser markings) to indicate the depth of insertion in order to assist the surgeon in placing the tissue anchor correctly within the tissue.

## Claims

1. A tissue anchor (1) comprising:
a first portion (5a) and a second portion (5b);
a sharp point at a leading end (6p) on the first portion (5a);
a resilient device (7x) between the first portion (5a) and the second portion (5b),
wherein the resilient device (7x) biases the first portion (5a) and the second portion (5b) apart from one another in order to urge them into an open configuration;
and **characterised in that** the anchor (1) further comprises at least one barb (6b) associated with the sharp point at the leading end (6p) and pointing in a direction away from the leading end, such that the anchor (1) is configured to resist withdrawal from the tissue in the open configuration.

2. A tissue anchor (1) according to claim 1, wherein the sharp point at the leading end (6p) is configured to penetrate dense tissue.

3. A tissue anchor (1) according to claim 1 or claim 2, wherein the first portion (5a) and the second portion (5b) are movable to a closed delivery configuration wherein the first portion (5a) and the second portion (5b) are urged towards one another against the bias of the resilient device (7x).

4. A tissue anchor (1) according to any preceding claim, wherein the at least one barb (6b) and the sharp point at the leading end (6p) are arranged to point in substantially opposite directions in the open configuration.

5. A tissue anchor (1) according to any preceding claim, wherein the first portion (5a) and the second portion (5b) are mirror image parts.

6. A tissue anchor (1) according to any preceding claim, wherein the first portion (5a) and the second portion (5b) each have a sharp point at the leading end (6p) and a corresponding barb (6b).

7. A tissue anchor (1) according to any preceding claim, wherein each of the first and second portions (5a, 5b) is in the form of a hook (6) with the leading end having a point (6p) for penetrating tissue, which point (6p) extends back to a rearward pointing barb (6b) at the trailing end.

8. A tissue anchor (1) according to any preceding claim, wherein the at least one barb (6b) has a greater lateral extent than the sharp leading end (6p).

9. A tissue anchor (1) according to any preceding claim, wherein the resilient device (7x) is a resilient wire.

10. A tissue anchor (1) according to any one of claims 1 to 8, wherein the resilient device (7x) is a piece of sprung plastic material.

11. A tissue anchor (1) according to any preceding claim, wherein the tissue anchor (1) is capable of at least a degree of rotational movement around an axis of insertion, so as to lodge the or each barb (6b) and the sharp point at the leading end (6p) within the tissue.

12. A tissue anchor (1) according to any preceding claim, wherein the resilient device (7x) is substantially teardrop shaped in the form of a loop.

13. A tissue anchor system comprising a tissue anchor (1) according to any preceding claim and a delivery sleeve (10) for biasing the tissue anchor (1) into the closed delivery configuration, wherein the diameter of the delivery sleeve (10) is less than the lateral extent of the barbs (6b) of the tissue anchor (1) when in the closed delivery configuration.

14. A tissue anchor system according to claim 13, wherein the delivery sleeve (10) has a throughbore for accommodating a suture (9), which is coupled to the anchor (1), within the throughbore.

15. A tissue anchor system comprising a tissue anchor (1) according to any of claims 1- to 12, and a recovery sleeve (12) adapted to accommodate at least a portion of the resilient device (7x), wherein the recovery sleeve (12)_is provided of larger diameter than the lateral extent of the barbs (6b) of the tissue anchor (1).

16. A tissue anchor system according to claim 15, wherein a leading edge of the recovery sleeve (12) is provided with cutting formations.

17. A tissue anchor system according to claim 15 or claim 16, wherein an internal surface of the recovery sleeve (12) is provided with an annular groove to accommodate the laterally outermost barbs (6b) on the tissue anchor (1).

18. A tissue anchor system according to any of claims 15 to 17, wherein the recovery sleeve (12) is provided with a throughbore for accommodating a delivery sleeve (10) therein.

## Patentansprüche

1. Eine Gewebeverankerung (1), die Folgendes beinhaltet:
einen ersten Abschnitt (5a) und einen zweiten Abschnitt (5b);
eine scharfe Spitze an einem Vorderende (6p) auf dem ersten Abschnitt (5a);
eine nachgiebige Vorrichtung (7x) zwischen dem ersten Abschnitt (5a) und dem zweiten Abschnitt (5b),
wobei die nachgiebige Vorrichtung (7x) den ersten Abschnitt (5a) und den zweiten Abschnitt (5b) auseinander vorspannt, um sie in eine offene Konfiguration zu drängen;
und **dadurch gekennzeichnet, dass** die Verankerung (1) ferner mindestens einen Widerhaken (6b), der mit der scharfen Spitze an dem Vorderende (6p) verbunden ist und in eine von dem Vorderende wegführende Richtung zeigt, beinhaltet, so dass die Verankerung (1) konfiguriert ist, um in der offenen Konfiguration einem Rückzug aus dem Gewebe zu widerstehen.

2. Gewebeverankerung (1) gemäß Anspruch 1, wobei die scharfe Spitze an dem Vorderende (6p) konfiguriert ist, um dichtes Gewebe zu durchdringen.

3. Gewebeverankerung (1) gemäß Anspruch 1 oder Anspruch 2, wobei der erste Abschnitt (5a) und der zweite Abschnitt (5b) in eine geschlossene Zuführungskonfiguration bewegbar sind, wobei der erste Abschnitt (5a) und der zweite Abschnitt (5b) gegen die Vorspannung der nachgiebigen Vorrichtung (7x) zueinander hingedrängt werden.

4. Gewebeverankerung (1) gemäß einem der vorhergehenden Ansprüche, wobei der mindestens eine Widerhaken (6b) und die scharfe Spitze an dem Vorderende (6p) angeordnet sind, um in der offenen Konfiguration in im Wesentlichen entgegengesetzte Richtungen zu zeigen.

5. Gewebeverankerung (1) gemäß einem der vorhergehenden Ansprüche, wobei der erste Abschnitt (5a) und der zweite Abschnitt (5b) spiegelbildliche Teile sind.

6. Gewebeverankerung (1) gemäß einem der vorhergehenden Ansprüche, wobei der erste Abschnitt (5a) und der zweite Abschnitt (5b) jeweils eine scharfe Spitze an dem Vorderende (6p) und einen entsprechenden Widerhaken (6b) aufweisen.

7. Gewebeverankerung (1) gemäß einem der vorhergehenden Ansprüche, wobei sowohl der erste als auch der zweite Abschnitt (5a, 5b) in Form eines Hakens (6) vorliegt, wobei das Vorderende einen Punkt (6p) zum Eindringen in Gewebe aufweist, wobei sich der Punkt (6p) zurück zu einem nach hinten zeigenden Widerhaken (6b) an dem nachfolgenden Ende erstreckt.

8. Gewebeverankerung (1) gemäß einem der vorhergehenden Ansprüche, wobei der mindestens eine Widerhaken (6b) eine größere laterale Erstreckung aufweist als das spitze Vorderende (6p).

9. Gewebeverankerung (1) gemäß einem der vorhergehenden Ansprüche, wobei die nachgiebige Vorrichtung (7x) ein nachgiebiger Draht ist.

10. Gewebeverankerung (1) gemäß einem der Ansprüche 1 bis 8, wobei die nachgiebige Vorrichtung (7x) ein Stück gefedertes Kunststoffmaterial ist.

11. Gewebeverankerung (1) gemäß einem der vorhergehenden Ansprüche, wobei die Gewebeverankerung (1) wenigstens zu einem Grad einer Drehbewegung um eine Einführungsachse imstande ist, um den oder jeden Widerhaken (6b) und die scharfe Spitze an dem Vorderende (6p) innerhalb des Gewebes zu befestigen.

12. Gewebeverankerung (1) gemäß einem der vorhergehenden Ansprüche, wobei die nachgiebige Vorrichtung (7x) im Wesentlichen tropfenförmig in Form einer Schleife ist.

13. Ein Gewebeverankerungssystem, das eine Gewebeverankerung (1) gemäß einem der vorhergehenden Ansprüche und eine Zuführungshülse (10) zum Vorspannen der Gewebeverankerung (1) in die geschlossene Zuführungskonfiguration beinhaltet, wobei der Durchmesser der Zuführungshülse (10) geringer ist als die laterale Erstreckung der Widerhaken (6b) der Gewebeverankerung (1), wenn diese sich in der geschlossenen Zuführungskonfiguration befindet.

14. Gewebeverankerungssystem gemäß Anspruch 13, wobei die Zuführungshülse (10) eine Durchgangsbohrung zum Unterbringen eines Nahtmaterials (9), das an die Verankerung (1) gekoppelt ist, innerhalb der Durchgangsbohrung aufweist.

15. Ein Gewebeverankerungssystem, das eine Gewebeverankerung (1) gemäß einem der Ansprüche 1 bis 12 und eine Rückholhülse (12), die angepasst ist, um mindestens einen Abschnitt der nachgiebigen Vorrichtung (7x) unterzubringen, beinhaltet, wobei die Rückholhülse (12) mit einem größeren Durchmesser versehen ist als die laterale Erstreckung der Widerhaken (6b) der Gewebeverankerung (1).

16. Gewebeverankerungssystem gemäß Anspruch 15, wobei eine Vorderkante der Rückholhülse (12) mit Einschnittformationen versehen ist.

17. Gewebeverankerungssystem gemäß Anspruch 15 oder Anspruch 16, wobei eine innere Oberfläche der Rückholhülse (12) mit einer ringförmigen Rille versehen ist, um die lateral äußersten Widerhaken (6b) auf der Gewebeverankerung (1) unterzubringen.

18. Gewebeverankerungssystem gemäß einem der Ansprüche 15 bis 17, wobei die Rückholhülse (12) mit einer Durchgangsbohrung zum Unterbringen einer Zuführungshülse (10) darin versehen ist.

## Revendications

1. Une ancre pour tissu (1) comprenant :
une première portion (5a) et une deuxième portion (5b) ;
une pointe acérée au niveau d'une extrémité d'attaque (6p) sur la première portion (5a) ;
un dispositif élastique (7x) entre la première portion (5a) et la deuxième portion (5b),
dans laquelle le dispositif élastique (7x) écarte par décalage la première portion (5a) et la deuxième portion (5b) l'une de l'autre afin de les pousser dans une configuration ouverte ;
et **caractérisée en ce que** l'ancre (1) comprend en outre au moins un ardillon (6b) associé à la pointe acérée au niveau de l'extrémité d'attaque (6p) et qui pointe dans une direction éloignée de l'extrémité d'attaque, de telle sorte que l'ancre (1) est configurée pour résister à son retrait du tissu dans la configuration ouverte.

2. Une ancre pour tissu (1) selon la revendication 1, où la pointe acérée au niveau de l'extrémité d'attaque (6p) est configurée pour pénétrer dans du tissu dense.

3. Une ancre pour tissu (1) selon la revendication 1 ou la revendication 2, où la première portion (5a) et la deuxième portion (5b) peuvent être déplacées vers une configuration d'administration fermée où la première portion (5a) et la deuxième portion (5b) sont poussées l'une vers l'autre contre le décalage du dispositif élastique (7x).

4. Une ancre pour tissu (1) selon n'importe quelle revendication précédente, où cet au moins un ardillon (6b) et la pointe acérée au niveau de l'extrémité d'attaque (6p) sont agencés pour pointer dans des directions substantiellement opposées dans la configuration ouverte.

5. Une ancre pour tissu (1) selon n'importe quelle revendication précédente, où la première portion (5a) et la deuxième portion (5b) sont des parties symétriques.

6. Une ancre pour tissu (1) selon n'importe quelle revendication précédente, où la première portion (5a) et la deuxième portion (5b) ont chacune une pointe acérée au niveau de l'extrémité d'attaque (6p) et un ardillon correspondant (6b).

7. Une ancre pour tissu (1) selon n'importe quelle revendication précédente, où chacune des première et deuxième portions (5a, 5b) est sous la forme d'un crochet (6), l'extrémité d'attaque ayant une pointe (6p) destinée à pénétrer dans du tissu, laquelle pointe (6p) s'étend à l'arrière jusqu' à un ardillon pointant vers l'arrière (6b) au niveau de l'extrémité arrière.

8. Une ancre pour tissu (1) selon n'importe quelle revendication précédente, où cet au moins un ardillon (6b) a une étendue latérale supérieure à l'extrémité d'attaque acérée (6p).

9. Une ancre pour tissu (1) selon n'importe quelle revendication précédente, où le dispositif élastique (7x) est un fil élastique.

10. Une ancre pour tissu (1) selon n'importe laquelle des revendications 1 à 8, où le dispositif élastique (7x) est un morceau de matière plastique à ressort.

11. Une ancre pour tissu (1) selon n'importe quelle revendication précédente, où l'ancre pour tissu (1) est capable d'au moins un degré de déplacement rotatif autour d'un axe d'insertion, de façon à loger le ou chaque ardillon (6b) et la pointe acérée au niveau de l'extrémité d'attaque (6p) au sein du tissu.

12. Une ancre pour tissu (1) selon n'importe quelle revendication précédente, où le dispositif élastique (7x) est substantiellement conformé en poire sous la forme d'une boucle.

13. Un système d'ancre pour tissu comprenant une ancre pour tissu (1) selon n'importe quelle revendication précédente et un manchon d'administration (10) destiné à décaler l'ancre pour tissu (1) dans la configuration de dégagement fermée, où le diamètre du manchon de dégagement (10) est inférieur à l'étendue latérale des ardillons (6b) de l'ancre pour tissu (1) lorsqu'elle se trouve en configuration de dégagement fermée.

14. Un système d'ancre pour tissu selon la revendication 13, où le manchon de dégagement (10) a un trou débouchant destiné à recevoir une suture (9), laquelle est couplée à l'ancre (1), au sein du trou débouchant.

15. Un système d'ancre pour tissu comprenant une ancre pour tissu (1) selon n'importe lesquelles des revendications 1 à 12, et un manchon de récupération (12) adapté pour recevoir au moins une portion du dispositif élastique (7x), où le manchon de récupération (12) est prévu d'un diamètre plus important que l'étendue latérale des ardillons (6b) de l'ancre pour tissu (1).

16. Un système d'ancre pour tissu selon la revendication 15, où un bord d'attaque du manchon de récupération (12) est pourvu de formations coupantes.

17. Un système d'ancre pour tissu selon la revendication 15 ou la revendication 16, où une surface interne du manchon de récupération (12) est pourvue d'une rainure annulaire pour recevoir les ardillons latéralement les plus externes (6b) sur l'ancre pour tissu (1).

18. Un système d'ancre pour tissu selon n'importe lesquelles des revendications 15 à 17, où le manchon de récupération (12) est pourvu d'un trou débouchant destiné à y recevoir un manchon de dégagement (10).
